# FASCICULE DE BREVET EUROPEEN

(11) **EP 0 709 423 B1**
(45) Date de publication et mention de la délivrance du brevet: **09.04.1997**
(21) Numéro de dépôt: 95402112.7
(22) Date de dépôt: 19.09.1995
(51) Int. Cl.: C08G 85/00

(54) **Polymère de 5,6-dihydroxyindole, leur procédé de préparation et compositions les comprenant**
Polymere aus 5,6-Dihydroxyindol, Verfahren zu ihrer Herstellung und diese enthaltende Zusammensetzungen
5,6-dihydroxyindole polymers, their process of preparation and compositions comprising the same

(30) Priorité: 25.10.1994 FR 9412743
(43) Date de publication de la demande: 01.05.1996
(73) Titulaire: L'OREAL, 75008 Paris (FR)
(72) Inventeur: Marrot, Laurent, F-93190 Livry-Gargan (FR)
(74) Mandataire: Dodin, Catherine

(56) Documents cités:
- US-A- 4 859 668
- TETRAHEDRON, (INCL TETRAHEDRON REPORTS), vol. 43, no. 22, 1987 OXFORD GB, pages 5351-5356, M.D'ISCHIA ET ALL. 'Sulfhydryl compounds in melanogenesis. Part I. Reaction of cysteine and glutathione with 5,6-dihydroxyindoles.'

## Description

L'invention a pour objet de nouveaux composés se présentant sous la forme de polymères de 5,6-dihydroxyindole, leur procédé de préparation et les compositions cosmétiques les comprenant.

Les composés mélaniques sont généralement connus sous la forme de pigments insolubles. Il s'agit plus particulièrement des pigments qui sont à l'origine de la coloration des cheveux, de la peau ou des poils d'origine humaine ou animale.
Ils peuvent être préparés par synthèse, en particulier par oxydation de dérivés indoliques tels que plus particulièrement le 5,6-dihydroxyindole.
Leur utilisation en cosmétique est très recherchée du fait qu'ils se rapprochent beaucoup des pigments existant à l'état naturel.
Ces pigments mélaniques sont généralement insolubles dans l'eau et dans la plupart des solvants couramment utilisés en cosmétique. Du fait de leur insolubilité, la mise en oeuvre de ces pigments peut poser certains problèmes comme l'utilisation d'agents dispersants de manière à obtenir des compositions cosmétiques présentant une bonne homogénéité.
Certains auteurs tels que ORLOW, OSBER, et PAWELECK [Pigment Cell Research 5, pages 113 à 121, (1992)] ont décrit une méthode pour obtenir un pigment mélanique soluble dans l'eau à un pH supérieur à 5, à partir du 5,6-dihydroxyindole 2-carboxylique acide (DHICA) seul ou en mélange avec du 5,6-dihydroxyindole (5,6-DHI). Ces pigments mélaniques solubles sont bien sûr de mise en oeuvre plus aisée que des pigments insolubles puisqu'ils ne nécessitent pas l'utilisation d'agents dispersants, cependant ils ne conservent pas toujours une solubilité satisfaisante après lyophilisation et remise en solution.
D'autre part, dans la demande de brevet WO 92-16189, il est décrit la préparation d'un pigment mélanique soluble en solution aqueuse à un pH variant entre 5 et 9 à partir de DHICA éventuellement associé à du 5,6-DHI et/ou à des composés soufrés. Selon ce document, la présence de composés soufrés permet d'obtenir des pigments solubles ayant des couleurs plus variées. Cependant ces composés, bien qu'ils soient solubles, ne possèdent pas un pouvoir colorant satisfaisant.

La présente invention a pour but de résoudre ces problèmes et de proposer un nouveau polymère de 5,6-dihydroxyindole qui soit soluble en milieu aqueux ou hydroalcoolique, tout en possédant un pouvoir colorant satisfaisant.

La présente invention a donc pour premier objet un composé se présentant sous la forme d'un polymère de 5,6-dihydroxyindole substitué par des groupements hydrophiles liés audit polymère par un résidu soufré, susceptible d'être obtenu par un procédé dans lequel :
- on prépare une solution aqueuse et/ou hydroalcoolique, ayant un pH au moins égal à 5, d'un composé soufré à groupements hydrophiles choisi parmi les composés comportant au moins un atome de soufre et ne pouvant conduire à la cyclisation en benzothiazine par réaction avec le 5,6-dihydroxyindole,
- on ajoute à cette solution du 5,6-dihydroxyindole et on laisse la réaction se poursuivre jusqu'à obtention du composé final recherché,
la quantité de composé soufré utilisée et le nombre de groupements hydrophiles étant choisis de manière à ce que ledit composé final présenté une solubilité en milieu aqueux et/ou hydroalcoolique, comprise entre 50 et 100 mg/ml.

On a constaté que le polymère selon l'invention est soluble, en particulier, dans un milieu aqueux et/ou hydroalcoolique présentant un pH au plus égal à 7, c'est-à-dire un pH neutre, voire un pH acide, et qu'il conserve une bonne solubilité dans l'eau et/ou en milieu hydroalcoolique même après lyophilisation et stockage. On a également constaté que ce polymère soluble de 5,6-dihydroxyindole est de couleur noire et présente une bonne absorption dans le visible. Il possède un pouvoir colorant comparable, voire supérieur, à celui des polymères mélaniques solubles de l'état de la technique. Il est de plus très stable au stockage après lyophilisation et peut être remis en solution.

La présente invention a également pour objet un procédé de préparation d'un composé tel que défini ci-dessus, dans lequel :
. on prépare une solution aqueuse et/ou hydroalcoolique, ayant un pH au moins égal à 5, d'un composé soufré à groupements hydrophiles, choisi parmi les composés comportant au moins un atome de soufre et ne couvant conduire à la cyclisation en benzothiazine par réaction avec le 5,6-dihydroxyindole,
. on ajoute à cette solution du 5,6-dihydroxyindole et on laisse la réaction se poursuivre jusqu'à obtention du composé final recherché, la quantité de composé soufré utilisée et le nombre de groupements hydrophiles étant choisie de manière à ce que le composé final obtenu présente une solubilité en milieu aqueux et/ou hydroalcoolique, comprise entre 50 et 100 mg/ml.

Le composé selon l'invention se présente donc sous la forme d'un polymère de 5,6-dihydroxyindole substitué par des groupements hydrophiles liés audit polymère par un résidu soufré.

Ce composé est constitué par le produit de réaction entre le 5,6-dihydroxyindole et au moins un composé soufré à groupements hydrophiles.

En effet les benzothiazines sont des composés intermédiaires dans la synthèse des phaeomélanines (mélanines de couleur rousse qui sont photo sensibilisantes pour la peau). Selon l'invention, la cyclisation en benzothiazine ne peut avoir lieu, éliminant ainsi les risques d'une possible photosensibilisation de la peau.
Parmi ces composés soufrés, on peut citer en particulier les peptides soufrés à groupements hydrophiles tels que le glutathion réduit, ou bien la N-acétylcystéine. La quantité de composé soufré présent lors de la réaction est choisie de manière à permettre l'obtention d'un composé final soluble en milieux aqueux et/ou hydroalcoolique. La concentration en composé soufré utilisée est de préférence comprise entre 0,001 et 1 % en poids environ par rapport au poids total du milieu réactionnel.
Le composé soufré est de préférence mis préalablement en solution aqueuse ou hydroalcoolique, voire organique, ladite solution ayant un pH au moins égal à 5. En effet, on a constaté que lorsque le pH est inférieur à 5, le 5,6-dihydroxyindole ne peut réagir avec le composé soufré.

Pour ajuster le pH, on peut employer des agents acidifiants ou alcalinisants classiques tels que l'acide chlorhydrique et la soude.
Le pH de la solution est de préférence compris entre 5 et 10, préférentiellement entre 6 et 8.

La concentration en 5,6-dihydroxyindole utilisée peut varier dans de larges proportions et de préférence entre 0,001 et 10 % en poids environ par rapport au poids total du milieu réactionnel.
Le 5,6-dihydroxyindole est de préférence ajouté sous forme de poudre, et la réaction est de préférence effectuée à une température supérieure à 0°C, par exemple comprise entre 30 et 40°C. De façon encore plus préférentielle, la température de réaction est comprise entre 36 et 38 °C.
Le temps de réaction nécessaire à l'obtention du composé final soluble peut être de l'ordre de 6 à 72 heures.
Le milieu réactionnel peut en outre contenir des agents régulateurs de pH encore appelés "tampons" parmi lesquels on peut citer le phosphate de sodium.

On obtient ainsi un composé mélanique se présentant sous la forme d'un polymère de 5,6-dihydroxyindole substitué par des groupements hydrophiles liés audit polymère par un résidu soufré, le nombre de groupements hydrophiles étant choisi de manière à ce que ledit composé soit soluble en milieu aqueux ou hydroalcoolique.
La solubilité de ce composé mélanique dans ledit milieu est comprise entre 50 et 100 mg/ml.

On peut utiliser ce composé tel quel sous forme d'une solution aqueuse ou hydroalcoolique, ou bien le concentrer, ou encore le sécher, par exemple par lyophilisation de manière à obtenir un produit pulvérulent que l'on peut utiliser dans les compositions cosmétiques.

La présente invention a également pour objets l'utilisation des composés mélaniques tels que définis ci-dessus à titre de colorant, en particulier dans des compositions cosmétiques, et les compositions obtenues.

Ces composés mélaniques solubles peuvent en effet être incorporés, tels quels, concentrés et/ou après lyophilisation, dans différents types de compositions cosmétiques telles que par exemple des compositions pour le maquillage de la peau, des cils ou des sourcils, des compositions pour la protection de l'épiderme humain contre le rayonnement UV et/ou des compositions pour la coloration des cheveux.
Dans ces compositions, la concentration des composés mélaniques peut varier de 0,001 à 5% en poids par rapport au poids total de la composition.

Lorsque les compositions sont utilisées pour le maquillage de la peau, des cils ou des sourcils, par exemple en tant que crème de traitement de l'épiderme, fond de teint, bâton de rouge à lèvres, fard à paupières, fard à joues, ligneur encore appelés "eye-liner" ou mascara, elles peuvent se présenter sous forme solide, liquide ou pâteuse, anhydre ou aqueuse, comme des émulsions huile-dans-eau ou eau-dans-huile, des dispersions, des lotions plus ou moins épaissies, des sticks ou des poudres. Ces compositions présentent l'avantage d'être parfaitement stables puisqu'il n'est pas nécessaire de disperser le composé mélanique du fait de sa solubilité.
Lorsque les compositions sont utilisées pour la protection de l'épiderme humain contre le rayonnement UV, elles constituent des compositions dites solaires et elles se présentent généralement sous forme d'émulsions telles que crèmes et laits, de pommades, de gels, de bâtonnets solides ou de mousses aérosols. Les émulsions peuvent en outre contenir des agents tensioactifs anioniques, cationiques, non ioniques, amphotères, zwittérioniques et leurs mélanges.
Lorsque les compositions sont utilisées pour la coloration des cheveux, elles peuvent se présenter sous forme de shampooing, de lotion, de gel ou de composition à rincer, à appliquer avant ou après shampooing, avant, pendant ou après permanente ou défrisage, de lotion ou gel coiffants ou traitants, de lotion ou gel pour le Brushing ou la mise en plis, de laque pour cheveux, de composition de permanente ou de défrisage.
Les compositions de maquillage et les compositions solaires peuvent également contenir en outre au moins un adjuvant choisi parmi les corps gras, les solvants organiques, les silicones, les épaississants, les adoucissants, les filtres solaires, les agents anti-mousse, les agents hydratants, les parfums, les conservateurs, les agents antioxydants, les charges, les pigments, les agents séquestrants, les agents de traitement tels que les polymères anioniques, cationiques, non ioniques, amphotères ou leurs mélanges, les propulseurs et les agents alcalinisants ou acidifiants.
Parmi les corps gras, on peut citer les huiles, les cires, les acides gras, les alcools gras, la vaseline, la paraffine et la lanoline, hydrogénée ou acétylée.
Les huiles peuvent en particulier être choisies parmi les huiles animales, végétales, minérales ou de synthèse et notamment l'huile de palme hydrogénée, l'huile de ricin hydrogénée, l'huile de vaseline, l'huile de paraffine et l'huile de Purcellin. Les cires peuvent en particulier être choisies parmi les cires animales, végétales, minérales ou de synthèse; on peut en particulier citer les cires d'abeilles, les cires de Carnauba, de Candellila, de canne à sucre, du Japon, les ozokérites, la cire de Montan, les cires microcristallines, les paraffines et les cires de silicones.
Parmi les pigments, on peut citer les pigments nacrés et/ou nacrants qui permettent de faire varier les colorations susceptibles d'être obtenues avec les composés mélaniques solubles de l'invention ou d'augmenter la protection de la peau vis-à-vis du rayonnement ultraviolet. Dans ce dernier cas, on peut utiliser des pigments métalliques tels que les oxydes de titane, de zinc, de cérium ou de zirconium.

On peut également associer le composé selon l'invention à un composé tel que la DHA dans des compositions cosmétiques. En effet, si les groupements hydrophiles du composé selon l'invention portent des amines libres, il est possible qu'ils favorisent l'établissement de pontages entre la peau et/ou les cheveux via des composés tels que la DHA. On peut ainsi obtenir des compositions de teinture ou coloration de la peau et/ou du cheveux.

Les exemples qui suivent sont destinés à illustrer l'invention sans pour autant en limiter la portée.

### Exemple 1

On dispose 10 mM de glutathion réduit dans une solution tampon à pH 7,5. On ajuste le pH à 7 par ajout d'hydroxyde de sodium. On ajoute 10 mM de 5,6-DHI en poudre et l'on agite le mélange réactionnel pendant 48 à 72 heures à 37°C.
Le composé obtenu n'est pas décantable par centrifugation à 15000 tr/min pendant 10 minutes. Il peut être filtré sur pores de 0,22µm et pénètre dans un gel d'agarose à 1%.
On lyophilise le composé filtré selon les techniques connues.
Immédiatement après lyophilisation, ce composé se resolubilise dans l'eau lorsqu'il est présent à une concentration de 50 mg/ml.
Après stockage pendant 6 semaines à 4°C, le composé lyophilisé est toujours soluble dans l'eau pour une concentration de 50 mg/ml.

### Exemple 2

On dispose 5 mM de glutathion réduit dans une solution tampon à pH 7,5. On ajuste le pH à 7 par ajout d'hydroxyde de sodium. On ajoute 10 mM de 5,6-DHI en poudre et l'on agite le mélange réactionnel pendant 48 à 72 heures à 37°C.
Le composé obtenu n'est pas décantable par centrifugation à 15000 tr/min pendant 10 minutes. Il peut être filtré sur pores de 0,22µm et pénètre dans un gel d'agarose à 1%.
On lyophilise le composé filtré selon les techniques connues.
Immédiatement après lyophilisation, ce composé se resolubilise dans l'eau pour une concentration de 50 mg/ml.

### Exemple 3

On dispose 15 mM de glutathion réduit dans une solution tampon à pH 7,5. On ajuste le pH à 7 par ajout d'hydroxyde de sodium. On ajoute 20 mM de 5,6-DHI en poudre et l'on agite le mélange réactionnel pendant 48 à 72 heures à 37°C.
Le composé obtenu n'est pas décantable par centrifugation à 15000 tr/min pendant 10 minutes. Il peut être filtré sur pores de 0,22µm et pénètre dans un gel d'agarose à 1%.
On lyophilise le composé filtré selon les techniques connues. Immédiatement après lyophilisation, ce composé se resolubilise dans l'eau pour une concentration de 50 mg/ml.

### Exemple 4

On mesure la densité optique à 475 nm et 600 nm de différents composés selon l'invention, solubilisés dans l'eau. On effectue cette mesure pour plusieurs concentrations.
On détermine ainsi le coefficient massique d'absorption qui est la pente de la droite obtenue expérimentalement.
On obtient les résultats suivants:
a) pour le composé de l'exemple 1
   ε475 nm = 6 lg⁻¹cm⁻¹ environ
   ε600 nm = 3,5 lg⁻¹cm⁻¹ environ
b) pour le composé de l'exemple 2
   les valeurs du coefficient massique obtenus sont du même ordre que pour le composé 1
   ε475 nm = 6 lg⁻¹cm⁻¹ environ
   ε600 nm = 3,5 lg⁻¹cm⁻¹ environ
c) pour le composé de l'exemple 3, les valeurs sont plus faibles :
   ε475 nm = 3,7 lg⁻¹cm⁻¹ environ
   ε600 nm = 2,2 lg⁻¹cm⁻¹ environ
Ceci est vraisemblablement dû à une incorporation plus importante de glutathion dans le polymère de 5,6-DHI: pour une même masse, la proportion de résidus indoliques responsables de l'absorption dans le visible est moindre.
Plus l'absorption dans le visible sera important, plus le composé mélanique obtenu sera de couleur foncée et plus son pouvoir colorant sera bon.

### Exemple 5

On dispose des quantités précises de composé obtenu selon l'exemple 1 dans de l'eau à 37°C, pendant 30 minutes.
On divise l'échantillon en deux parties dont l'une est soumise à filtration sur pores de 0,45 µm ou 0,22 µm.
Les deux parties de l'échantillon sont ensuite ramenées à une concentration de 1 mg/ml et laissées à nouveau à 37°C, afin de solubiliser les éventuelles particules résidentes.
On compare les densités optiques à 600 nm et 475 nm.
On considère que la filtrabilité reflète la solubilité à concentration choisie.

On constate que :
. pour une concentration de 50 mg/ml, il n'y a pas de rétention du composé par filtration.
. pour une concentration de 100 mg/ml, on récupère 95% du composé après filtration sur pores de 0,45 µm. On obtient un résultat identique si l'on remplace la filtration par une centrifugation à 15 000 tr/min pendant 10 minutes.
Après filtration sur pores de 0,22 µm, on récupère 87% du composé.

On peut donc estimer que ce composé présente une limite de solubilité dans l'eau de l'ordre de 100 mg/ml.

### Exemple 6

On compare l'absorption lumineuse du composé obtenu à l'exemple 1, avec un composé selon l'état de la technique obtenue par polymérisation de DHICA à une concentration de 10 mM.
Les spectres d'absorption sont enregistrés pour une solution à 50 µM en résidus indoliques dans l'eau.
On obtient les résultats suivants:

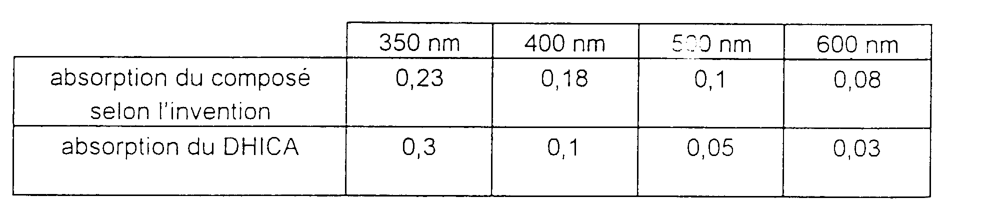

On effectue les mêmes mesures pour un composé selon l'invention obtenu à partir de 5 mM de 5,6-DHI et 5 mM de glutathion, comparé à un composé obtenu à partir de 5 mM de DHICA.
On obtient les résultats suivants:

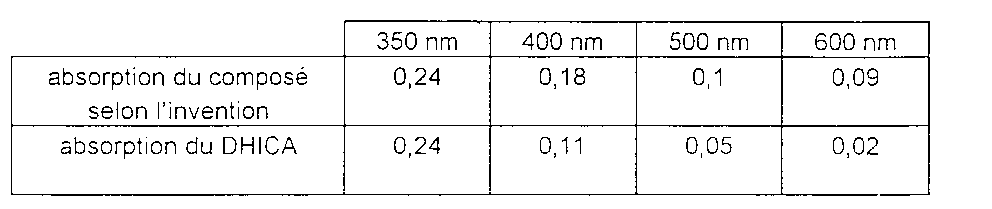

On constate donc que les composés selon l'invention absorbent d'avantage dans le visible que les composés de l'état de la technique.
Ceci se traduit par l'obtention d'une couleur plus soutenue, plus intense, lorsque l'on utilise une composition comprenant le composé selon l'invention.

## Revendications

1. Composé se présentant sous la forme d'un polymère de 5,6-dihydroxyindole substitué par des groupements hydrophiles liés audit polymère par un résidu soufré, susceptible d'être obtenu par un procédé dans lequel :
- on prépare une solution aqueuse et/ou hydroalcoolique, ayant un pH au moins égal à 5, d'un composé soufré à groupements hydrophiles choisi parmi les composés comportant au moins un atome de soufre et ne pouvant conduire à la cyclisation en benzothiazine par réaction avec le 5,6-dihydroxyindole,
- on ajoute à cette solution du 5,6-dihydroxyindole et on laisse la réaction se poursuivre jusqu'à obtention du composé final recherché,
la quantité de composé soufré utilisée et le nombre de groupements hydrophiles étant choisis de manière à ce que ledit composé final présente une solubilité en milieu aqueux et/ou hydroalcoolique, comprise entre 50 et 100 mg/ml.

2. Composé selon la revendication 1, présentant une solubilité comprise entre 50 et 100 mg/ml, dans un milieux aqueux et/ou hydroalcoolique ayant un pH au plus égal à 7.

3. Composé selon l'une des revendications précédentes, dans lequel le composé soufré est choisi parmi la N-acétylcystéine et les peptides soufrés à groupements hydrophiles.

4. Composé selon l'une des revendications précédentes, dans lequel le composé soufré est le glutathion réduit.

5. Composé selon l'une des revendications précédentes, se présentant sous forme d'une solution aqueuse ou hydroalcoolique, sous forme concentrée ou sous forme pulvérulente.

6. Procédé de préparation d'un composé tel que défini dans l'une quelconque des revendications précédentes, dans lequel :
. on prépare une solution aqueuse et/ou hydroalcoolique, ayant un pH au moins égal à 5, d'un composé soufré à groupements hydrophiles, choisi parmi les composés comportant au moins un atome de soufre et ne pouvant conduire à la cyclisation en benzothiazine par réaction avec le 5,6-dihydroxyindole,
. on ajoute à cette solution du 5,6-dihydroxyindole et on laisse la réaction se poursuivre jusqu'à obtention du composé final recherché, la quantité de composé soufré utilisée et le nombre de groupements hydrophiles étant choisis de manière à ce que ledit composé final présente une solubilité en milieu aqueux et/ou hydroalcoolique, comprise entre 50 et 100 mg/ml.

7. Procédé selon la revendication 6, dans lequel le composé soufré est choisi parmi la N-acétylcystéine et les peptides soufrés à groupements hydrophiles.

8. Procédé selon l'une quelconque des revendications 6 à 7, dans lequel le composé soufré est le glutathion réduit.

9. Procédé selon l'une quelconque des revendications 6 à 8, dans lequel la concentration en composés soufrés à groupements hydrophiles utilisée est comprise entre 0,001 et 1 % en poids par rapport au poids total du milieu réactionnel.

10. Procédé selon l'une quelconque des revendications 6 à 9, dans lequel la concentration en 5,6-dihydroxyindole utilisée est comprise entre 0,001 et 10 % en poids par rapport au poids total du milieu réactionnel.

11. Procédé selon l'une quelconque des revendications 6 à 10, dans lequel le 5,6-dihydroxyindole est ajouté sous forme de poudre.

12. Procédé selon l'une quelconque des revendications 6 à 11, dans lequel la réaction est effectuée à une température supérieure à 0°C, de préférence entre 30 et 40°C.

13. Procédé selon l'une quelconque des revendications 6 à 12, dans lequel le pH du milieu réactionnel est compris entre 5 et 10, de préférence entre 6 et 8.

14. Utilisation du composé selon l'une quelconque des revendications 1 à 5, en tant que colorant dans la préparation de compositions cosmétiques.

15. Composition cosmétique comprenant au moins un composé tel que défini dans l'une quelconque des revendications 1 à 5.

16. Composition selon la revendication 15, dans laquelle la concentration du composé est comprise entre 0,001 et 5 % en poids par rapport au poids total de la composition.

17. Composition selon l'une des revendications 15 à 16, se présentant sous forme d'un produit de maquillage de la peau, des cils ou des sourcils, d'un produit de protection de l'épiderme humain contre le rayonnement U.V. et/ou d'un produit pour la coloration des cheveux et/ou de la peau.

18. Composition selon l'une des revendications 15 à 17, se présentant sous forme d'une émulsion, d'une dispersion, d'une lotion plus ou moins épaissie, d'un stick, d'une poudre, d'une pâte anhydre ou non ou d'une mousse, aérosol ou non.

19. Composition selon l'une des revendications 15 à 18, comprenant en outre de la DHA (dihydroxyacétone).

## Patentansprüche

1. Verbindungen, die in Form eines Polymers von 5,6-Dihydroxyindol vorliegen, das mit hydrophilen Gruppen substituiert ist, die an das Polymer über eine schwefelhaltige Gruppe gebunden sind, die durch ein Verfahren mit folgenden Schritten erhältlich sind:
- Herstellung einer wäßrigen und/oder wäßrig-alkoholischen Lösung einer schwefelhaltigen Verbindung mit hydrophilen Gruppen, die unter den Verbindungen ausgewählt sind, die mindestens ein Schwefelatom aufweisen und durch Reaktion mit 5,6-Dihydroxyindol nicht zu einer Cyclisierung zu Benzothiazin führen können, wobei die Lösung einen pH-Wert von mindestens 5 aufweist,
- Zusatz von 5,6-Dihydroxyindol zu dieser Lösung und Umsetzung bis zur Herstellung der gewünschten Endverbindung,
wobei die verwendete Menge der schwefelhaltigen Verbindung und die Zahl der hydrophilen Gruppen so ausgewählt ist, daß die am Ende vorliegende Verbindung eine Löslichkeit in wäßrigem und/oder wäßrig-alkoholischem Medium von 50 bis 100 mg/ml aufweist.

2. Verbindungen nach Anspruch 1, die in einem wäßrigen und wäßrig-alkoholischen Medium mit einem pH-Wert von höchstens 7 eine Löslichkeit im Bereich von 50 bis 100 mg/ml aufweisen.

3. Verbindungen nach einem der vorhergehenden Ansprüche, wobei die schwefelhaltige Verbindung unter N-Acetylcystein und den schwefelhaltigen Peptiden mit hydrophilen Gruppen ausgewählt ist.

4. Verbindungen nach einem der vorhergehenden Ansprüche, wobei die schwefelhaltige Verbindung reduziertes Glutathion ist.

5. Verbindungen nach einem der vorhergehenden Ansprüche, die in Form einer wäßrigen oder wäßrig-alkoholischen Lösung, in konzentrierter Form oder pulverförmig vorliegen.

6. Verfahren zur Herstellung einer Verbindung nach einem der vorhergehenden Ansprüche, mit folgenden Schritten:
. Herstellung einer wäßrigen und/oder wäßrig-alkoholischen Lösung einer schwefelhaltigen Verbindung mit hydrophilen Gruppen, die unter den Verbindungen ausgewählt ist, die mindestens ein Schwefelatom aufweisen und durch Reaktion mit 5,6-Dihydroxyindol nicht zur Cyclisierung zu Benzothiazin führen können, wobei die Lösung einen pH-Wert von mindestens 5 aufweist,
. Zusatz von 5,6-Dihydroxyindol zu dieser Lösung und Reaktion bis zur Herstellung der gewünschten Endverbindung,
wobei der verwendete Mengenanteil der schwefelhaltigen Verbindung und die Zahl der hydrophilen Gruppen so ausgewählt ist, daß die am Ende vorliegende Verbindung eine Löslichkeit in wäßrigem und/oder wäßrig-alkoholischem Medium von 50 bis 100 mg/ml aufweist.

7. Verfahren nach Anspruch 6, wobei die schwefelhaltige Verbindung unter N-Acetylcystein und den schwefelhaltigen Peptiden mit hydrophilen Gruppen ausgewählt ist.

8. Verfahren nach einem der Ansprüche 6 bis 7, wobei die schwefelhaltige Verbindung reduziertes Glutathion ist.

9. Verfahren nach einem der Ansprüche 6 bis 8, wobei die verwendete Konzentration der schwefelhaltigen Verbindungen mit hydrophilen Gruppen im Bereich von 0,01 bis 1 Gew.-%, bezogen auf das Gesamtgewicht des Reaktionsmediums, liegt.

10. Verfahren nach einem der Ansprüche 6 bis 9, wobei die verwendete Konzentration von 5,6-Dihydroxyindol im Bereich von 0,001 bis 10 Gew.-%, bezogen auf das Gesamtgewicht des Reaktionsmediums, liegt.

11. Verfahren nach einem der Ansprüche 6 bis 10, wobei das 5,6-Dihydroxyindol in Form von Pulver zugegeben wird.

12. Verfahren nach einem der Ansprüche 6 bis 11, wobei die Reaktion bei einer Temperatur über 0 °C und vorzugsweise von 30 bis 40 °C durchgeführt wird.

13. Verfahren nach einem der Ansprüche 6 bis 12, wobei der pH-Wert des Reaktionsmediums im Bereich von 5 bis 10 und vorzugsweise von 6 bis 8 liegt.

14. Verwendung einer Verbindung nach einem der Ansprüche 1 bis 5 als Färbemittel zur Herstellung von kosmetischen Zusammensetzungen.

15. Kosmetische Zusammensetzung, die mindestens eine Verbindung nach einem der Ansprüche 1 bis 5 enthält.

16. Zusammensetzung nach Anspruch 15, wobei die Konzentration der Verbindung im Bereich von 0,001 bis 5 Gew.-%, bezogen auf das Gesamtgewicht der Zusammensetzung, liegt.

17. Zusammensetzung nach einem der Ansprüche 15 bis 16, die in Form eines Produkts zum Schminken der Haut, der Wimpern oder der Augenbrauen, als Produkt zum Schutz der menschlichen Epidermis gegen UV-strahlung und/oder als Produkt zum Färben der Haare und/oder der Haut vorliegt.

18. Zusammensetzung nach einem der Ansprüche 15 bis 17, die in Form einer Emulsion, Dispersion, mehr oder weniger dickflüssigen Lotion, als Stift, Pulver, ggf. wasserfreier Paste oder als Aerosolschaum oder Schaum vorliegt.

19. Zusammensetzung nach einem der Ansprüche 15 bis 18, die ferner DHA (Dihydroxyaceton) enthält.

## Claims

1. Compound in the form of a 5,6-dihydroxyindole polymer substituted with hydrophilic groups attached to the said polymer via a sulphur-containing residue, capable of being obtained by a process in which:
. an aqueous and/or aqueous-alcoholic solution, having a pH at least equal to 5, of a sulphur-containing compound carrying hydrophilic groups, chosen from compounds which contain at least one sulphur atom and which cannot lead to cyclization to benzothiazine by reaction with 5,6-dihydroxyindole, is prepared,
. 5,6-dihydroxyindole is added to this solution and the reaction is allowed to continue until the final compound sought is obtained, the amount of sulphur-containing compound used and the number of hydrophilic groups being chosen so that the said final compound has a solubility of between 50 and 100 mg/ml in an aqueous and/or aqueous-alcoholic medium.

2. Compound according to Claim 1, having a solubility of between 50 and 100 mg/ml in an aqueous and/or aqueous-alcoholic medium having a pH of not more than 7.

3. Compound according to one of the preceding claims, in which the sulphur-containing compound is chosen from N-acetylcysteine and sulphur-containing peptides carrying hydrophilic groups.

4. Compound according to one of the preceding claims, in which the sulphur-containing compound is reduced glutathione.

5. Compound according to one of the preceding claims, which is in the form of an aqueous or aqueous-alcoholic solution, in concentrated form or in pulverulent form.

6. Process for the preparation of a compound as defined in any one of the preceding claims, in which:
. an aqueous and/or aqueous-alcoholic solution, having a pH at least equal to 5, of a sulphur-containing compound carrying hydrophilic groups, chosen from compounds which contain at least one sulphur atom and which cannot lead to cyclization to benzothiazine by reaction with 5,6-dihydroxyindole, is prepared,
. 5,6-dihydroxyindole is added to this solution and the reaction is allowed to continue until the final compound sought is obtained, the amount of sulphur-containing compound used and the number of hydrophilic groups being chosen so that the said final compound has a solubility of between 50 and 100 mg/ml in an aqueous and/or aqueous-alcoholic medium.

7. Process according to Claim 6, in which the sulphur-containing compound is chosen from N-acetylcysteine and sulphur-containing peptides carrying hydrophilic groups.

8. Process according to either of Claims 6 and 7, in which the sulphur-containing compound is reduced glutathione.

9. Process according to any one of Claims 6 to 8, in which the concentration of sulphur-containing compounds carrying hydrophilic groups used is between 0.001 and 1 % by weight relative to the total weight of the reaction medium.

10. Process according to any one of Claims 6 to 9, in which the concentration of 5,6-dihydroxyindole used is between 0.001 and 10 % by weight relative to the total weight of the reaction medium.

11. Process according to any one of Claims 6 to 10, in which the 5,6-dihydroxyindole is added in powder form.

12. Process according to any one of Claims 6 to 11, in which the reaction is carried out at a temperature above 0°C, preferably between 30 and 40°C.

13. Process according to any one of Claims 6 to 12, in which the pH of the reaction medium is between 5 and 10, preferably between 6 and 8.

14. Use of the compound according to any one of Claims 1 to 5, as a dye in the preparation of cosmetic compositions.

15. Cosmetic composition comprising at least one compound as defined in any one of Claims 1 to 5.

16. Composition according to Claim 15, in which the concentration of the compound is between 0.001 and 5 % by weight relative to the total weight of the composition.

17. Composition according to either of Claims 15 and 16, which is in the form of a make-up product for the skin, the eyelashes or the eyebrows, of a product for protection of the human epidermis against UV radiation and/or of a product for colouring the hair and/or the skin.

18. Composition according to one of Claims 15 to 17, which is in the form of an emulsion, a dispersion, a lotion which is more or less thickened, a stick, a powder, a paste, which may or may not be anhydrous, or a foam, which may or may not be an aerosol foam.

19. Composition according to one of Claims 15 to 18, which also comprises DHA (dihydroxyacetone).
